Europäisches Patentamt

European Patent Office

Office européen des brevets

(11) Veröffentlichungsnummer: **0 313 851**
**A2**

## (12) EUROPÄISCHE PATENTANMELDUNG

(21) Anmeldenummer: 88116102.0

(22) Anmeldetag: 29.09.88

(51) Int. Cl.⁴ **A61J 1/00**

(30) Priorität: 28.10.87 DE 3736487

(43) Veröffentlichungstag der Anmeldung:
03.05.89 Patentblatt 89/18

(84) Benannte Vertragsstaaten:
AT BE CH DE ES FR GB GR IT LI LU NL SE

(71) Anmelder: **B. Braun Melsungen AG**
**Carl-Braun Strasse**
**D-3508 Melsungen(DE)**

(72) Erfinder: **Haindl, Hans-Günther**
**Forstgarten 22**
**D-3508 Melsungen(DE)**

(74) Vertreter: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Flexibler Mehrkammerbehälter.**

(57) Bei einem flexiblen Mehrkammerbehälter zur Sterilisation, Lagerung und Mischung von Mischinfusionslösungen mit einer aus Folienblättern (11, 12) gebildeten Beutelanordnung, dient zur Unterteilung in Kammern eine von außen öffenbare Verschlußvorrichtung (20), die aus einem Kernstab (25) und einem diesen klauenartig umspannenden stabförmigen Aufsteckteil (27) besteht, die die Folienblätter (11, 12) eines Beutelkörpers zwischen sich einklemmen.

Eine solche einfach handhabbare Verschlußvorrichtung hält erhöhten Innendrücken bei der Sterilisation der Anordnung stand und erlaubt eine preiswerte Herstellung des Mehrkammerbehälters.

FIG.2

EP 0 313 851 A2

## Flexibler Mehrkammerbehälter

Die Erfindung bezieht sich auf einen flexiblen Mehrkammerbehälter zur Sterilisation, Lagerung und Mischung von Mischinfusionslösungen mit einem aus zwei Folienblättern gebildeten Beutelkörper, der in mehrere Kammern unterteilt ist und an der Trennlinie zwischen den Kammern eine von außen öffenbare Verschlußvorrichtung aufweist. Ein solcher Mehrkammerbehälter ist aus der DE 32 38 649 C2 bekannt.

Bei manchen Anwendungsfällen medizinischer Infusionen werden unterschiedliche Substanzen verwendet, die, weil sie keine stabile, sterilisationsfähige Mischung bilden, getrennt aufbewahrt werden müssen und erst kurz vor der Infusion gemischt werden dürfen. Um zu vermeiden, daß solche Substanzen in getrennten Einzelbehältern gelagert werden müssen und über ein aufwendiges System von Leitungen, Tropfkammern und Ventilen erst in dem zum Patienten führenden Leitungsstrang gemischt werden können (US 4 447 230), sind Mehrkammerbehälter entwickelt worden. Ein bekannter derartiger Mehrkammerbehälter (US 3 858 580) besteht aus einem steifen Zylinder, der von einem gummielastischen Stopfen in zwei Kammern unterteilt ist. Der Stopfen wird mit Hilfe eines von außen in den Zylinder einführbaren Stabes gekippt und der Inhalt beider Kammern kann durch Schütteln vermischt werden, bevor er in die Infusionsleitung gelangt. Bei der Handhabung dieser Vorrichtung ergeben sich durch den von außen einzuführenden Stab Sterilitätsprobleme. Außerdem ist die Mischaktivität des Schüttelns bei größeren Anteilen jeder Infusionsmittelkomponente nicht immer ausreichend.

Um diesem Mangel zu begegnen, werden flexible Mehrkammerbeutel benutzt, die beispielsweise für die parenterale Ernährung vor der Infusion Aminosäure-Lösung einerseits und Zuckerlösung andererseits in Kammern voneinander getrennt enthalten. Als Verschlußvorrichtung zwischen den Kammern dienen Brechröhrchen, die in einem Kammertrennsteg des Beutels eingeschweißt sind und nach dem Zerbrechen von außen ein Mischlumen freigeben (DE 77 19 528 U1). Abgesehen davon, daß die durch das Brechröhrchen hergestellte Verbindung zwischen zwei Kammern klein ist, so daß es ausgiebiger Mischaktivitäten durch mehrfaches Durchkneten der Beutelanordnung bedarf, um die Infusion infusionsfertig zu machen, hat diese Anordnung den Nachteil, daß beim Zerbrechen des Brechröhrchens Partikel freigesetzt werden, die in die Infusionsleitung gelangen können. Ein anderer bekannter Mehrkammerbehälter in Form einer flexiblen Beutelanordnung, die aus an Trennwänden zusammenhängenden Einzelbeuteln besteht und eingangs erwähnt ist (DE 32 38 649 C2), weist als Verschlußvorrichtung eine in der Trennwand vorgegebene, haftverschweißte Reißlinie auf, an der von außen zugängliche Reißgriffe befestigt sind. Derartige Haftverschweißungen haben den Nachteil, daß eine Hitzesterilisation des Inhaltes der einzelnen Beutel problematisch ist, da durch den innerhalb des Beutels während der Sterilisation auftretenden Druck die Trennwand vorzeitig aufreißen kann, wenn keine sorgfältige Druckkompensation vorgenommen worden ist. Um dieser Gefahr vorzubeugen, ist die Reißlinie mit öffenbaren Verschlußvorrichtungen verstärkt, die eine zusätzliche Steigerung der Herstellungskosten für solche flexiblen Mehrkammerbehälter hervorrufen.

Der Erfindung liegt die Aufgabe zugrunde, einen flexiblen Mehrkammerbehälter durch eine Verschlußvorrichtung zu verbessern, die erhöhten Innendrücken in den Kammern zuverlässig standhält und die eine preiswerte Herstellung der Beutelanordnung erlaubt.

Diese Aufgabe wird bei einem Mehrkammerbehälter der eingangs genannten Art erfindungsgemäß dadurch gelöst, daß die Verschlußvorrichtung aus einem Kernstab und einem diesen klauenartig umspannenden stabförmigen Aufsteckteil besteht, die die Folienblätter des Beutelkörpers zwischen sich einklemmen.

Die klemmend wirksame Verschlußvorrichtung kann an jeder gewünschten Stelle zwischen zwei Enden eines aus zwei Folienblättern preiswert hergestellten einfachen Beutelkörpers angesetzt werden, um diesen in zwei getrennte Kammern zu unterteilen. Mit Hilfe mehrerer Verschlußvorrichtungen kann der Beutelkörper in beliebig viele getrennte Kammern unterteilt werden, indem an den gewünschten Stellen ein gerader klauenartiger Aufsteckteil auf einen geraden Kernstab radial aufgesteckt oder axial aufgeschoben wird, der in eine Schlaufe oder Falte der beiden Folienblätter eingelegt ist. Kernstab und Aufsteckteil können sich über die gesamte Höhe des Beutelkörpers erstrecken, so daß bei Freigabe der Verschlußvorrichtung der gesamte lichte Querschnitt des Beutelkörpers offen ist. Um die in den beiden Kammern befindlichen Lösungen zu mischen, wird einfach der Kernstab axial aus dem stabförmigen Aufsteckteil herausgezogen, wodurch beide Teile voneinander freikommen und die eingeklemmte Schlaufe des Beutelkörpers sich glättet, so daß die beiden Folienblätter sich voneinander trennen und die beiden Lösungen das gesamte Lumen des Beutelkörpers ausfüllen. Mit geringen Knetbearbeitungen des Beutelkörpers zur Durchmischung der Lösungen kann die Infusion infusionsfertig gemacht werden.Die Verschlußvorrichtung kann sich auch an eine über einen Teil der Beutelhöhe verlaufende Schweißnaht axial anschließen, wenn aus bestimmten Gründen die Versteifung des Beutelkörpers über seine gesamte Höhe nicht gewünscht wird und ein verkleinerter Übergang zwischen zwei Kammern zulässig ist. Die Abdichtwirkung der Verschlußvorrichtung wird durch erhöhte Innendrücke infolge einer Hitzesterilisation nicht beeinträchtigt, weil Kernstab und Aufsteckteil so fest ineinanderpassen, daß die Innendrücke sich nicht auf den Spaltbereich zwischen beiden Teilen auswirken und die gegenseitige Klemmposition nur durch Krafteinwirkung von außen aufgehoben werden kann.

Die Beutelanordnung läßt sich preiswert in einer Form-, Füll- und Siegelmaschine herstellen. In diesem Falle wird zunächst ein offener, viereckiger Beutelkörper durch Verschweißung der beiden Folienblätter an drei Rändern geformt, dann wird der untere Teil des Beutelkörpers befüllt und durch die angesetzte Verschlußvorrichtung zugeklemmt. Anschließend wird der Teil des Beutelkörpers über der Verschlußvorrichtung gefüllt und sodann wird der obere vierte Rand der Folienblätter versiegelt. Durch dieses rationelle Vorgehen wird die Herstellung des mit unterschiedlichen Lösungen gefüllten Mehrkammerbehälters verbilligt.

Die Profile des Kernstabes und des stabförmigen Aufsteckteiles lassen sich in unterschiedlicher Weise gestalten. Vorzugsweise hat der Kernstab runden, im wesentlichen kreisförmigen, Querschnitt, der von der komplementären Klaue des Aufsteckteils um mehr als 180° umfaßt wird. Die beiden Folienblätter verlassen die Klemmöffnung paarweise nach entgegengesetzten Seiten der Verschlußvorrichtung und diese steht als wulstförmige Verdickung über die Seitenfläche des einen Folienblattes vor. Der Kernstab kann aus Kunststoff oder Metall, vorzugsweise Aluminium, hergestellt und massiv oder hohl sein. Ein hohler Kernstab ist vorteilhafterweise an einer Längsseite offen, so daß auch er zwei Schenkel erhält, die eine gewisse radiale Klemmkraft gegen den ihn umgebenden, komplementär gestalteten Aufsteckteil ausüben und die Festklemmung der beiden Folienblätter verstärken.

An einem Ende des Kernstabes und/oder des Aufsteckteiles ist ein Griffstück angeordnet. Dieses steht zweckmäßig über das Ende des jeweils anderen Teiles nach außen vor und erleichtert die Trennung von Kernstab und Aufsteckteil zur Öffnung des Durchlasses zwischen zwei Kammern des Beutelkörpers.

Erfindungsgemäß ist das Verfahren zur Herstellung des Mehrkammerbehälters dadurch gekennzeichnet, daß gegen das eine Folienblatt des Beutelkörpers ein schmaler Streifen und gegen das andere Folienblatt ein breiterer Streifen aus biegsamem Metall angelegt wird, daß dem breiteren Streifen eine Werkzeugpatrize und dem schmaleren Streifen eine klauenartig schließbare Werkzeugmatrize zugeordnet wird und daß durch Bewegung der Werkzeugpatrize in Richtung der Werkzeugmatrize und Schließung der Werkzeugmatrize der breitere Streifen als Aufsteckteil um den schmaleren Streifen als Kernstab herumgeformt wird. Werkzeugpatrize und Werkzeugmatrize sind zweckmäßigerweise so ausgebildet, daß der Kernstab und der Aufsteckteil im Querschnitt Teilkreisform erhalten und die beiden Folienblätter fest zwischen sich einspannen, so daß eine gute Abdichtung der von beiden Seiten der Klemmanordnung abgehenden Kammern gegeneinander erreicht wird.

In der Zeichnung sind Ausführungsbeispiele der Erfindung schematisch dargestellt.

Es zeigen:

Fig. 1 eine Draufsicht auf einen Beutelkörper mit zwei Kammern,

Fig. 2 eine Ausführungsform der Verschlußvorrichtung im Querschnitt,

Fig. 3 eine weitere Ausführungsform der Verschlußvorrichtung im Querschnitt,

Fig. 4 eine dritte Möglichkeit der Ausbildung der Verschlußvorrichtung im Querschnitt und

Fig. 5 einen Querschnitt von Maschinenteilen zur Herstellung der Verschlußvorrichtung nach Figur 4.

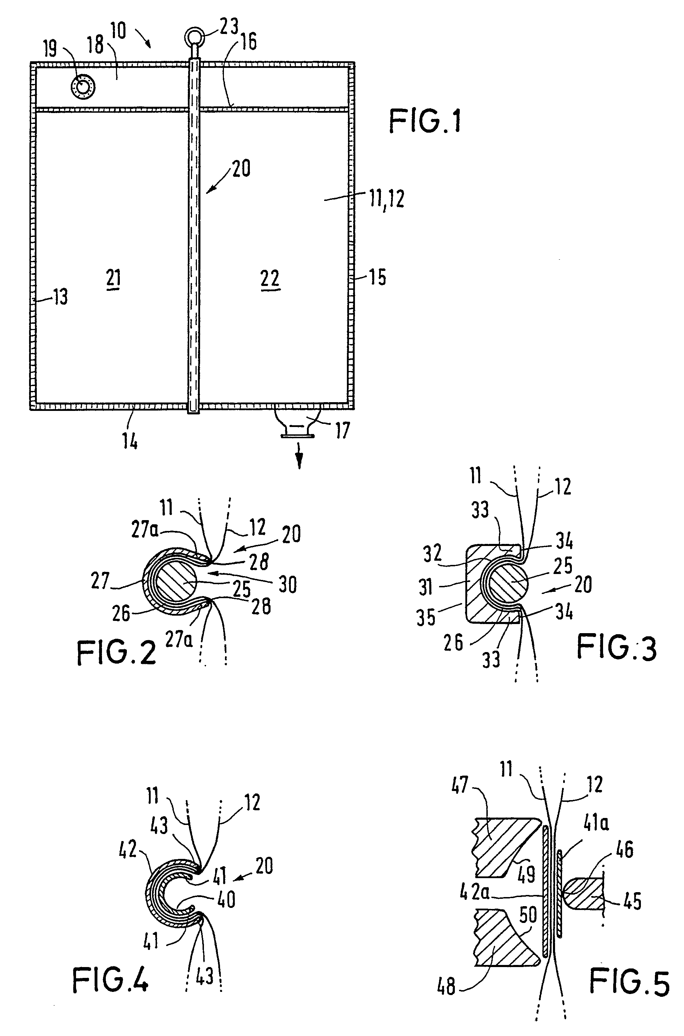

Der in Figur 1 gezeigte Doppelkammerbehälter zur Sterilisation, Lagerung und Mischung von Mischinfusionslösungen, z.B. für die parenterale Ernährung, besteht aus einem einteiligen Beutelkörper 10, der aus zwei aufeinanderliegenden Folienblättern 11, 12 aus flexiblem, transparentem Kunststoff hergestellt ist. Die beiden Folienblätter 11 und 12 sind bei dem in Figur 1 gezeigten Beispiel viereckig. Sie können jedoch auch beliebige geeignete andere Form aufweisen. Die Folienblättern 11, 12 sind an ihren drei geraden Rändern 13, 14, 15 miteinander verschweißt. An einem Ende des Randes 14 ist ein Auslauf 17 angebracht, der mit dem Inneren des Beutelkörpers 10 in Verbindung steht und an eine nicht gezeichnete Infusionsvorrichtung anschließbar ist. Der obere Rand 16 ist mit einem Verstärkungsstreifen 18 verschweißt, in dem an dem dem Auslauf 17 gegenüberliegenden Ende eine Aufhängeöse 19 ausgebildet ist. Die Diametralanordnung vom Auslauf 17 und Aufhängeöse 19 bewirkt eine Schräghängung des Beutelkörpers 20 und infolgedessen eine verbesserte Entleerung seines Innenraumes bei Durchführung einer Infusion.

Der Beutelkörper 10 wird mit Hilfe der erfindungsgemäßen Verschlußvorrichtung 20 in zwei Kammern 21, 22 unterteilt, deren Volumen je nach Ansetzen der Verschlußvorrichtung 20 in bezug auf die Breite des Beutelkörpers 10 beliebig gewählt werden kann. Jede Kammer 21, 22 wird mit Hilfe eines Ansatzstückes mit einer Komponente der Mischinfusionslösung befüllt. Beispielsweise kann die Kammer 21 Aminosäure-Lösung enthalten, während sich in der Kammer 22 Glucoselösung befindet. Nach der Befüllung der Kammern 21, 22 werden die Ansatzstücke entfernt und der Rand 16 wird zugeschweißt. Anschließend wird die gesamte Anordnung sterilisiert, was ohne Dichtigkeitsrisiko durch erhöhte Innendrücke vor sich geht.

Alternativ dazu besteht die Möglichkeit, die Fertigung des Mehrkammerbehälters in den Prozeß einer Form-, Füll- und Siegelmaschine einzufügen. Hierbei wird zunächst der an dem Rand 15 offene Beutelkörper 10 geformt, dann wird die nach unten weisende Kammer 21 befüllt, es wird die Verschlußvorrichtung 20 angeklemmt und die obere Kammer 22 gefüllt. Anschließend wird der Rand 15 versiegelt.

Die Verschlußvorrichtung 20 ist in den Figuren 2, 3 und 4 in verschiedenen Variationen abgebildet.

Gemäß Figur 2 besteht sie aus einem geraden zylindrischen Kernstab 25 mit kreisförmigem Querschnitt, der aus Kunststoff mit einer gewissen Steifigkeit und Festigkeit hergestellt ist. Der Kernstab 25 wird an der für die Aufteilung des Beutelkörpers 10 in die beiden Kammern 21, 22 gewünschten Stelle gegen das eine Folienblatt, z.B. das Folienblatt 11 so angelegt, daß er sich zwischen den beiden Rändern 14 und 18 rechtwinklig zu diesen erstreckt. Der Kernstab 25 wird zur Bildung einer Schlaufe oder Falte 26 des Beutelkörpers 10 in diesen hineingedrückt, so daß ihn die beiden Folienblätter 11 und 12 längs einer geraden Trennlinie umschlingen. Sodann wird ein stabförmiger zylindrischer Aufsteckteil 27, der aus Aluminium oder Kunststoff gefertigt sein kann und im Querschnitt angenäherte Hufeisenform hat, auf den folienumschlungenen Kernstab 25 aufgeschoben oder radial aufgedrückt. Der innere Klauenbereich des Aufsteckteiles 27 ist dem zu umspannenden Kernstabumfang so angepaßt, daß sich eine Klemmwirkung ergibt, die ausreicht, um die beiden Folienblätter 11 und 12 zwischen Kernstab 25 und Aufsteckteil 27 festzuspannen und die Kammern 21, 22 gegeneinander abzudichten. Die federelastischen Schenkel 27a des Aufsteckteils 27 umfassen den Kernstab 25 um mehr als 180° und ragen tangential über den Kreisumfang des Kernstabes 25 hinaus. Die Längsränder dieser Schenkel 27a begrenzen eine Längsöffnung 30 und sind glatt abgerundet, damit die um diese Enden bei 28 seitwärts abgelenkten Folienblätter 11, 12 bei Füllung der Kammern 21, 22 nicht beschädigt werden. Diese seitliche Ablenkung an den Stellen 28 bietet eine zusätzliche Abdichtung der beiden Kammern 21, 22 gegeneinander, weil die Folienblätter 11, 12 an den Längsrändern der Schenkel 27a im Füllzustand der Kammern 21, 22 fest gegeneinander gepreßt werden.

Der Kernstab 25 ist länger als der Beutelkörper 10 und trägt an einem Ende eine Grifföse 23. Die Länge des stabförmigen Aufsteckteiles 27 entspricht im wesentlichen derjenigen des Beutelkörpers 10 zwischen oberem und unterem Rand, so daß seine beiden Enden mit den Rändern 14 und 18 des Beutelkörpers 10 im wesentlichen bündig abschließen.

Zur Öffnung der beiden Kammern 21, 22 gegeneinander wird der Kernstab 25 mit Hilfe der Grifföse 23 aus dem Aufsteckteil 27 axial herausgezogen, die Umschlagfalte 26 streckt sich und rutscht aus der Längsöffnung 30 zwischen den beiden Schenkeln 27a heraus, so daß der Aufsteckteil 27 freikommt und der Durchgang zwischen den beiden Kammern 21, 22 von einem Ende des Beutelkörpers 10 zum anderen offen ist. Zur Mischung der beiden Infusionslösungen wird der einteilige Beutelkörper 10 durchgeknetet und der ungedrosselte Übergang zwischen den beiden Kammern 21, 22 führt zu einem raschen, sehr guten Mischergebnis der zu infundierenden Mischinfusionslösungen, die durch den Auslauf 17 infundiert werden.

Bei dem Beispiel der Figur 3 wird der Kernstab 25 mit kreisförmigem Querschnitt benutzt, um an beliebiger Stelle des Beutelkörpers 10 die beiden Folienblätter 11, 12 zu einer Falte 26 zu formen. Die Falte 26 wird zur Bildung eines stabförmigen Aufsteckteiles 31 mit Kunststoff umspritzt. Der Aufsteckteil 31 kann als rechteckiger Stab gestaltet sein, der eine nach einer Seite offene längsverlaufende Rille 32 mit teilkreisförmigem Querschnitt aufweist. Die die Rille 32 flankierenden parallelen Schenkel 33 des Aufsteckteiles 31 sind so lang, daß sie den Halbmesser des in die Rille 32 hineingedrückten Kernstabes 25 überragen. Die teilkreisförmige Wand der Rille 32 umfaßt den Umfang des Kernstabes 25 um mehr als 180°. Die Längsränder 34 der beiden Schenkel 33 liegen auf gemeinsamer Ebene parallel zur Rückenfläche 35 des Aufsteckteiles und sind ebenflächig oder abgerundet, damit die seitwärts abgelenkten Folienblätter 11, 12 nicht beschädigt werden.

Das Beispiel nach Figur 4 zeigt eine dritte Möglichkeit der Ausbildung der Verschlußvorrichtung 20, die mit Maschinenteilen gemäß Figur 5 herstellbar ist. Diese Verschlußvorrichtung 20 besteht aus einem teilkreisförmig gebogenen Kernstab 40, der an einer Längsseite offen ist, so daß zwei Klemmschenkel 41 entstehen und aus einem Aufsteckteil 42, der entsprechend dem Kernstab 40 gestaltet ist und diesen klemmend umgreift. Seine beiden Klemmschenkel 43 stehen über die Enden der Klemmschenkel 41 des Kernstabes 40 etwas vor und die Klemmkraft zwischen den Schenkelpaaren 41 bzw. 43 bewirkt eine ausgezeichnete Abdichtung zwischen den Kammern 21, 22 des Beutelkörpers 10. Die stabförmigen Klemmorgane 40, 42 sind aus einem schmalen Aluminiumstreifen 41a und einem breiteren Aluminiumstreifen 42a hergestellt, die kunststoffbeschichtet sein können und deren Längsränder abgerundet sind. Die Maschinenteile zur Verformung der beiden Metallstreifen 41a, 42a bestehen aus einer Backe oder Rolle 45, deren umfangsmäßige Stirnkante 46 abgerundet ist und aus einem gegenüberliegenden Paar von Backen oder Rollen 47, 48, deren Stirnkanten eine konkave Schrägprofilierung 49, 50 aufweist, die sich zu einem Halbrund ergänzen, das an die Außenfläche des Metallstreifens 42a profilgebend angreift, wenn die Backen oder Rollen 47, 48 sowohl gegeneinander als auch in Richtung der Backe oder Rolle 45 verschoben werden. Die Verformung der ebenen Metallstreifen 41a und 42a erfolgt nach ihrer Anlegung gegen das Folienblatt 12 einerseits und das Folienblatt 11 andererseits. Da keine Verbindung der Folienblätter 11, 12 mit den Metallstreifen vorgesehen ist, lassen sich auch bei dieser Verschlußvorrichtung 20 der Kernstab 40 und der Aufsteckteil 42 durch axiales Auseinanderziehen voneinander trennen, wenn eine Verbindung der Kammern 21, 22 gewünscht wird.

## Ansprüche

1. Flexibler Mehrkammerbehälter zur Sterilisation, Lagerung und Mischung von Mischinfusionslösungen mit einem aus zwei Folienblättern (11, 12) gebildeten Beutelkörper, der in mehrere Kammern (21, 22) unterteilt ist und an der Trennlinie zwischen den Kammern eine von außen öffenbare Verschlußvorrichtung (20) aufweist,
**dadurch gekennzeichnet,** daß die Verschlußvorrichtung (20) aus einem Kernstab (25) und einem diesen klauenartig umspannenden stabförmigen Aufsteckteil (27) besteht, die die Folienblätter (11, 12) des Beutelkörpers (10) zwischen sich einklemmen.

2. Mehrkammerbehälter nach Anspruch 1,
**dadurch gekennzeichnet,** daß der Kernstab (25;40) runden, vorzugsweise kreisförmigen, Querschnitt aufweist und daß der dazu komplementäre Aufsteckteil (27;31;42) den Kernstab (25;40) um mehr als 180° umfaßt.

3. Mehrkammerbehälter nach Anspruch 1 oder 2,
**dadurch gekennzeichnet,** daß der Kernstab (25) aus Kunststoff besteht.

4. Mehrkammerbehälter nach Anspruch 1 oder 2,
**dadurch gekennzeichnet,** daß der Kernstab (40) aus Metall, vorzugsweise Aluminium, besteht.

5. Mehrkammerbehälter nach einem der Ansprüche 1 bis 4,
**dadurch gekennzeichnet,** daß der Kernstab (40) hohl ist.

6. Mehrkammerbehälter nach einem der Ansprüche 1 bis 4,
**dadurch gekennzeichnet,** daß der Kernstab (25) massiv ist.

7. Mehrkammerbehälter nach einem der Ansprüche 1 bis 6,
**dadurch gekennzeichnet,** daß der Kernstab (25) umfangsmäßig geschlossen ist.

8. Mehrkammerbehälter nach Anspruch 5,
**dadurch gekennzeichnet,** daß der Kernstab (40) an einer Längsseite offen ist.

9. Mehrkammerbehälter nach einem der Ansprüche 1 bis 8,
**dadurch gekennzeichnet,** daß an einem Ende des Kernstabes (25) und/oder des Aufsteckteiles (27) ein Griffstück (23) angeordnet ist.

10. Verfahren zur Herstellung eines Mehrkammerbehälters nach einem der Ansprüche 1, 2, 4, 5 und 8,
**dadurch gekennzeichnet,** daß gegen das eine Folienblatt des Beutelkörpers ein schmaler Streifen und gegen das andere Folienblatt ein breiterer Streifen aus biegsamem Metall angelegt wird, daß dem breitere Streifen eine Werkzeugpatrize und dem schmaleren Streifen eine klauenartig schließbare Werkzeugmatrize zugeordnet wird und daß durch Bewegung der Werkzeugpatrize in Richtung der Werkzeugmatrize und Schließung der Werkzeugmatrize der breitere Streifen als Aufsteckteil um den schmaleren Streifen als Kernstab herumgeformt wird.

FIG.1

FIG.2

FIG.3

FIG.4

FIG.5